# EUROPÄISCHE PATENTANMELDUNG

(11) **EP 2 923 676 A1**
(43) Veröffentlichungstag der Anmeldung: **30.09.2015**
(21) Anmeldenummer: 14161594.8
(22) Anmeldetag: 25.03.2014
(51) Int. Cl.: A61F 2/28, A61B 17/80, A61F 2/30

(54) **Augenhöhlenabdeckgitter mit außerkonturfolgenden länglichen Ausnehmungen**

(71) Anmelder: Karl Leibinger Medizintechnik GmbH & Co. KG, 78570 Mühlheim/Donau (DE)
(72) Erfinder: Reinauer, Frank, 78570 Muehlheim (DE); Grigore, Anita, 78570 Muehlheim (DE)
(74) Vertreter: Winter, Brandl, Fürniss, Hübner, Röss, Kaiser, Polte - Partnerschaft mbB

(57) **Zusammenfassung**

Die Erfindung betrifft ein Augenhöhlenabdeckgitter (1) mit einem Stege (4) ausbildenden perforierten Hauptkörper, der eine äußere Abschlusskante (6) aufweist, wobei mehrere längliche, durch einen Steg (4) von der Abschlusskante (6) getrennte und dort direkt angrenzende erste Ausnehmungen (5, 8) zumindest einem Abschnitt der Abschlusskante (6) folgend angeordnet sind. Die Erfindung betrifft auch ein Verfahren zum Herstellen eines Augenhöhlenabdeckgitters (1), wobei unter Nutzung von individuellen Patientendaten eine Negativform geschaffen wird, dann ein vorgefertigtes Rohstück mit ersten und zweiten länglichen Ausnehmungen (5, 8, 9) auf die Negativform aufgelegt wird, dann unter Aufbringung von Kraft/Druck und/oder Wärme das Rohstück an die Negativform angepasst wird, und das abgeformte, fertige Augenhöhlenabdeckgitter (1) von der Negativform abgenommen/vereinzelt wird.

## Beschreibung

Die Erfindung betrifft ein Augenhöhlenabdeckgitter, nämlich ein "orbita mesh", mit einem stegausbildenden perforierten Hauptkörper, der eine äußere, normalerweise umlaufende Abschlusskante/Einfassung aufweist.

Das Gitter wird als Anordnung aus länglichen Teilen in regelmäßigen oder unregelmäßigen Abständen verstanden. Es hat eine netzartig ausgestaltete Flächenstruktur.

Aus dem Stand der Technik sind bereits Augenhöhlenabdeckgitter bekannt, wie beispielsweise aus der EP 1 965 735 B1. Dort wird ein Implantat zur Verwendung als Ersatz eines Orbitabodens eingesetzt. Das Implantat ist als Augenhöhlenabdeckgitter ausgestaltet, liegt also am Orbitaboden auf. So ein Implantat, wie ein "Mesh" bzw. Gitter kann auch zur seitlichen Orbitarekonstruktion verwendet werden. Es kann auch freitragend eingesetzt werden und muss nicht unbedingt am Boden aufliegen. In der besagten Druckschrift wird ein Implantat für die Verwendung als Ersatz eines Augenhöhlengrunds und optional auch einer medialen Augenhöhlenwandung in der Form einer einstückig vorgeformten Platte, die einen ersten Abschnitt, einen zweiten Abschnitt und einen dritten Abschnitt umfasst, vorgestellt, wobei der erste Abschnitt gemäß einem Augenhöhlengrund und der zweite Abschnitt gemäß einer medialen Seitenwandung geformt sind und der erste Abschnitt und der zweite Abschnitt an einer ersten vorbestimmten Linie anliegen, wobei der dritte Abschnitt zur Befestigung des Implantats am vorderen Augenhöhlenrand angeordnet ist, wobei als besonders herausgestellt ist, dass die erste vorbestimmte Linie eine in der besagten Druckschrift als Bruchlinie definiert ist, entlang welcher ein Arzt ein Segment leicht entfernen kann.

Gitterartig ausgebildete Platten sind auch in ähnlicher Form zum Einsatz an anderen Teilen des Körpers bekannt.

So offenbart beispielsweise die DE 197 46 396 A1 ein Gitter für die Fixierung von Knochenteilen oder für die Überbrückung von Knochenfehlstellen. Ein solches Gitter kann auch am Schädel eingesetzt werden. Letztlich wird in dieser deutschen Druckschrift ein Gitter zur Anwendung im Schädel- und Kieferbereich vorgeschlagen, bestehend aus biokompatiblen Materialien mit einer netzartigen Struktur und mit Ausnehmungen zur Aufnahme von Knochenschrauben, mit denen das Gitter am Knochen befestigbar ist.. Die Stege bilden mäanderförmige, durchgehende, periodische Stegreihen entlang der Hauptachse des Gitters.

Das Augenhöhlenabdeckgitter, also jene Vorrichtung, die zum In-Kontakt-Gelangen mit dem Orbitaboden vorgesehen ist, darf bei Anbringung am Knochen die Augapfelaufnahme nicht behindern. Jene Augapfelaufnahme ist jedoch nicht sphärisch, sondern erstreckt sich länglich, insbesondere s-förmig.

Die aus dem Stand der Technik bekannten Augenhöhlenabdeckgitter sind leider häufig zu groß, nicht angepasst an den jeweils zu behandelnden individuellen Schädelknochen und auch häufig schwierig anzupassen.

Es ist die Aufgabe der Erfindung, hier Abhilfe zu bieten und eine gute Ausgangsstruktur eines Augenhöhlenabdeckgitters dem Operateur zur Verfügung zu stellen, insbesondere ein solches Augenhöhlenabdeckgitter, was nicht zu groß ist, bereits vorangepasst an den jeweiligen zu behandelnden Schädel ist und einfach feinanpassbar ist. Ferner soll ein Verfahren vorgestellt werden, das ein einfaches Herstellen eines solchen Augenhöhlenabdeckgitters ermöglicht. Letztlich soll auch ein Verfahren vorgestellt werden, um schnell und präzise Verletzungen des Orbitabodens dauerhaft zu behandeln.

### Offenbarung der Erfindung

Diese Aufgabe wird erfindungsgemäß durch ein Augenhöhlenabdeckgitter der gattungsgemäßen Art dadurch gelöst, dass erfindungsgemäß mehrere längliche (nur) durch einen aus Material des Hauptkörpers gebildeten Steg von der Abschlusskante getrennte und dort direkt angrenzende erste Ausnehmungen/Leerräume zumindest einem Abschnitt der Abschlusskante folgend angeordnet sind.

Durch Wegschneiden von Stegen lässt sich die Größe des Orbitabodengitters/Augenhöhlenabdeckgitters einfach an die zu versorgende Stelle anpassen. Der Einsatz einer Konturhilfe zum Ankonturieren ist möglich und wünschenswert.

Unter einem "Folgen der Abschlusskante" wird hier ein paralleles Folgen verstanden, also das pro Abschnitt "in derselben Richtung ausgerichtet sein".

Vorteilhafte Ausführungsformen sind in den Unteransprüchen beansprucht und werden nachfolgend näher erläutert.

So ist es von Vorteil, wenn ein Augenhöhlenabdeckgitter dadurch weitergebildet wird, dass in Richtung des Zentrums des Gitters eine oder mehrere, längliche Ausnehmungen/Leerräume vorhanden sind, die die gleiche Ausrichtung wie die jeweils benachbarte/nächstgelegene erste Ausnehmung aufweist/aufweisen. Die gesamte, das Gitter ausbildende Platte, ergo der Hauptkörper, kann dann perforiert sein. Der Einsatz einer Titanlegierung als Material ist dabei von besonderem Vorteil in puncto Stabilität und Biokompatibilität.

Es ist auch von Vorteil, wenn die innersten, zweiten Ausnehmungen einer Bügeleisensohlenaußenkontur folgend angeordnet sind oder dreieckig ausgerichtet sind. Die innersten zweiten Ausnehmungen sind also besipielsweise bügeleisensohlenaußenkonturartig angeordnet. Sie laufen von einer Grundfläche gesehen geschwungen / konvex in Richtung einer Spitze zu.

Es ist zweckmäßig, wenn der Hauptkörper einen Erweiterungsbereich aufweist, in dem zwei erste Ausnehmungen in einem spitzen Winkel zueinander, etwa 40° bis etwa 70°, vorzugsweise ca. 45°, ca. 50° oder ca. 55° aufeinander zulaufend, angeordnet/ausgerichtet sind.

Ein vorteilhaftes Ausführungsbeispiel ist auch dadurch gekennzeichnet, dass wenigstens eine beispielsweise radial vom Hauptkörper abstehende und/oder am Außenumfang" vorhandene/abstehende Lasche, vorzugsweise mehrere Laschen, vorhanden ist/vorhanden sind, die zur Aufnahme von Befestigungsmitteln, wie einer oder mehreren Schrauben, mit einem oder mehreren vorzugsweise kreisrund ausgestalteten Löchern versehen ist/versehen sind. Unter Einsatz bewährter Befestigungsmittel lässt sich dann während der Operation einfach die Fixierung des Augenhöhlenabdeckgitters am Schädelknochen realisieren.

Wenn die im Hauptkörper vorhandenen ersten und zweiten Ausnehmungen überwiegen, also etwa mehr als 50% oder besser mehr als 80%, vorzugsweise 85% bis 95% aller im Hauptkörper enthaltenen Ausnehmungen, eine längliche Kontur aufweisen, so wird eine besonders gute Anpassbarkeit erreicht.

Dabei ist es von Vorteil, wenn ein an eine radial abstehende Lasche angrenzendes Rundloch zwischen zwei ersten Ausnehmungen vorhanden ist. Eine zusätzliche Befestigung im Bereich des Hauptkörpers zur Anbringung am Knochen lässt sich dann realisieren. Die Belastbarkeit des Augenhöhlenabdeckgitters erhöht sich dadurch.

Auch ist es von Vorteil, wenn die Länge der ersten oder zweiten Ausnehmung mehr als 2,5-mal so groß wie die möglichst gleichbleibende Breite ist, vorzugsweise mehr als 3-mal, 3,5-mal oder 4-mal so groß, aber kleiner als das Zehnfache der Breite. Die Belastbarkeit des Augenhöhlenabdeckgitters steht dann in einem guten Verhältnis zur Fluiddurchlässigkeit.

Wenn das Gitter eine mehrfach gekrümmte Raumform einnimmt, vorzugsweise in allen drei Raumrichtungen gekrümmt ist, beispielsweise einer S-Form (im Querschnitt) folgt, so kann ein abstandsloses Anbringen des Augenhöhlenabdeckgitters am Orbitaboden erreicht werden.

Damit die Spannungslinien gut verlaufen, ist es von Vorteil, wenn sich eine zweite Ausnehmung bis zu einem die Abschlusskante/Einfassung ausformenden Steg erstreckt.

Ferner ist es zweckmäßig, wenn die Stege und Ausnehmungen so angeordnet/ausgerichtet und beschaffen sind, dass die Außenmaße des Gitters, insbesondere des Hauptkörpers, auf eine individuelle Augenhöhle, vorzugsweise manuell, werkzeuglos oder unter Einsatz einer Zange anpassbar sind.

Die Anpassbarkeit wird erleichtert, wenn die Stege und Ausnehmungen zur Ausbildung von einer vorgeschlagenen imaginären Trennlinie angeordnet/ausgerichtet und beschaffen sind, etwa nach Art von Sollbruchstellen/Perforationslinien/Sollbruchlinien.

Wenn innerhalb der ersten und (auch) zweiten Ausnehmungen ein perforationsloser Mittelsteg vorhanden ist, der etwa im Zentrum des Gitters bzw. des Hauptkörpers angeordnet ist, so kann die Position des Auges besonders gut stabilisiert werden, insbesondere wenn dieser flächig wirkende Mittelsteg in Richtung Posterior ausgerichtet ist/eingesetzt ist.

Damit das Augenhöhlenabdeckgitter nicht zu dick aufträgt, ist es von Vorteil, wenn zumindest der Hauptkörper, vorzugsweise auch die Lasche(n) eine gleichbleibende Dicke, etwa von ca. 0,2 mm bis 0,5 mm, vorzugsweise ca. 0,4 mm aufweist/aufweisen. Der Hauptkörper und die Lasche können insbesondere als integrale Bestandteile einstückig ausgebildet sein. Der Hauptkörper und die Lasche(n) sind also als Monolithteil ausgebildet.

Die Erfindung betrifft letztlich auch ein Verfahren zum Herstellen eines Augenhöhlenabdeckgitters etwa der erfindungsgemäßen Art, wobei unter Nutzung von individuellen Patientendaten eine Negativform / Konturhilfe / patientenspezifisch ausgeformte Schablone geschaffen wird, wobei dann ein vorgefertigtes, beispielsweise platten- und/oder gitterartiges, flächiges und bedarfsweise ebenes Rohstück/Ausgangsbauteil/Urfomteil mit ersten und zweiten länglichen Ausnehmungen auf die Negativform aufgelegt wird, dann unter Aufbringung von Kraft/Druck und/oder Wärme das Rohstück an die Negativform angepasst wird und das abgeformte/fertige Augenhöhlenabdeckgitter von der Negativform abgenommen/vereinzelt wird.

Auch ist die Erfindung einsetzbar bei der medizinischen Behandlung einer beschädigten und/oder deformierten Augenhöhle (Orbita), im Schädel (Cranium) eines Säugetiers, wie eines Menschen, wobei unter Nutzung von CT- oder Röntgentechniken ein Modell des Schädels des Säugetiers, aber auch jedes anderen Tieres, errechnet wird, mit Hilfe dessen und unter Nutzung des erfindungsgemäßen Herstellverfahrens zur Herstellung des erfindungsgemäßen Augenhöhlenabdeckgitters, eine individuell auf das zu behandelnde Säugetier angepasstes fertiges Augenhöhlenabdeckgitter geschaffen wird, das dann am Schädelknochen des zu behandelnden Säugetieres befestigt wird.

Die Erfindung wird nachfolgend mit Hilfe einer Zeichnung näher erläutert. Dabei ist ein erstes Ausführungsbeispiel wiedergegeben. Es zeigen:
- Fig. 1: eine Vorderansicht auf ein erfindungsgemäßes Augenhöhlenabdeckgitter /"orbita mesh",
- Fig. 2: eine Seitenansicht auf das Augenhöhlenabdeckgitter aus Fig. 1,
- Fig. 3: eine Seitenansicht von rechts auf das Augenhöhlenabdeckgitter aus Fig. 1,
- Fig. 4: eine Unteransicht des Augenhöhlenabdeckgitters aus Fig. 1,
- Fig. 5: eine Draufsicht auf das Augenhöhlenabdeckgitter aus Fig. 1,
- Fig. 6: eine Rückansicht auf das Augenhöhlenabdeckgitter aus Fig. 1,
- Fig. 7: das Vorhalten einer bogenförmigen Schnittlinie zum Erleichtern des Anpassens des Augenhöhlenabdeckgitters der Fig. 1 bis 6 durch Entfernung eines medialen Flügels im Rahmen einer Perforationslinie,
- Fig. 8: eine weitere Variante zum Vorhalten von Schnittmöglichkeiten an einem Posteriorabschnitt mit (Neben-)Schnittlinien, die zueinander parallel angeordnet sind und eine Hauptschnittlinie kreuzen,
- Fig. 9: das Vorhalten von Perforationslinien zum teilweisen Entfernen des medialen Flügels,
- Fig. 10: eine mediale Flügelperforationslinie und eine posterior gelegene Verkleinerungslinie, die sichelförmig ausgebildet sind,
- Fig. 11: eine asymmetrische Schnittlinie am orbitabodenseitigen Ende kombiniert mit einer Medialflügelentfernungslinie, und
- Fig. 12: eine Variante einer Schnittlinienausbildung zum teilweisen Abschneiden der Medialflügelwandung und eines Posteriorsegmentabschnittes.

Die Figuren sind schematischer Natur und dienen nur dem Verständnis der Erfindung. Die Merkmale der einzelnen Ausführungsbeispiele können untereinander ausgetauscht und kombiniert werden.

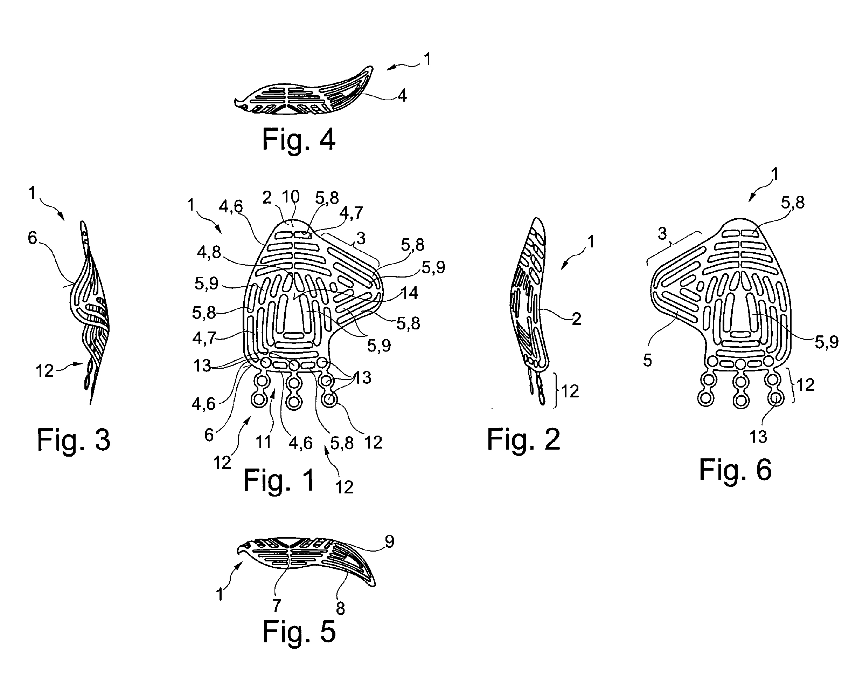

In Fig. 1 ist ein erfindungsgemäßes Augenhöhlenabdeckgitter 1 dargestellt. Das Augenhöhlenabdeckgitter 1 wird geometrisch durch einen Hauptkörper 2 bestimmt, der einen Medialflügel 3 aufweist. Der Hauptkörper 2 bildet Stege 4 aus, die zueinander durch Ausnehmungen/Leerräume 5 voneinander beabstandet sind. Der äußerste Steg 4 formt eine umlaufende Abschlusskante 6 aus, die auch als Einfassung bezeichnet werden kann. Die nicht auf der Umfangskante endenden Stege werden auch als innere Stege 7 bezeichnet.

Die Vielzahl von Ausnehmungen 5 wird unterschieden in erste Ausnehmungen 8 und zweite Ausnehmungen 9. Die Ausnehmungen 8 und 9 sind ausnahmslos länglich, teilweise auch kurvig ausgebildet. Sie haben eine gleichbleibende Breite und sind an ihren Enden abgerundet. Der Hauptkörper 2 weist eine Spitze 10 auf, dem gegenüberliegend ein abgeflachtes Ende 11 ausgebildet ist. Dort ragen drei Laschen 12 nach außen, hier also radial ab. Sie stehen auf der Abschlusskante 6 senkrecht. Jede Lasche 12 weist zwei kreisrunde Löcher 13 auf. Zwischen zwei dem abgeflachten Ende 11 nächstgelegenen ersten Ausnehmungen 8 ist ebenso ein solches Loch 13 vorgesehen. Auch benachbart zu diesem ist unter Zwischenschaltung von jeweils einer länglichen ersten Ausnehmung 8 je ein weiteres Loch 13 ausgebildet.

Im Inneren des Hauptkörpers 2, von fünf zweiten Ausnehmungen 9 umgeben, ist eine bügeleisensohlenartige Struktur vorhanden, die einen Mittelsteg 14 ausformt. Der Mittelsteg 14 hat also angenähert die Form eines Dreiecks mit an den Ecken vorhandenen Abrundungen, wobei zumindest an den Ecken Stege 4 anschließen.

Im Medialflügel 3 sind die ersten Ausnehmungen 8 und die zweiten Ausnehmungen 9 zueinander im spitzen Winkel aufeinander zulaufend ausgerichtet.

Die Figuren 2 bis 6 ermöglichen einen räumlichen Eindruck der in allen Raumrichtungen gebogenen Ausformung des Augenhöhlenabdeckgitters 1.

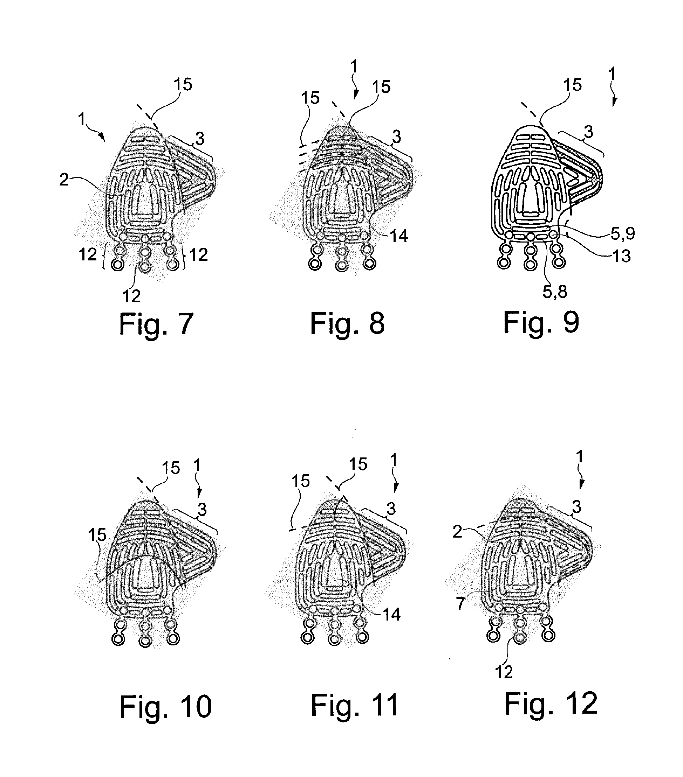

In den Figuren 7 bis 12 ist die stoffliche, geometrische und insbesondere kombinatorische Ausgestaltung der Stege 4 und Ausnehmungen 5 zueinander zum Ausformen von einer imaginären Trennlinie, etwa nach Art einer Sollbruchstellen/Perforationslinien/Sollbruchlinien 15, dargestellt.

In Fig. 8 kreuzen sich einzelne Perforationslinien 15, wohingegen andere zueinander parallel beabstandet bleiben und einer Kurvenform folgen. Der Medialflügel 3 bildet einen Erweiterungsbereich aus.

### Bezugszeichenliste

- 1: Augenhöhlenabdeckgitter
- 2: Hauptkörper
- 3: Medialflügel
- 4: Steg
- 5: Ausnehmung/Leerraum
- 6: Abschlusskante/Einfassung
- 7: innerer Steg
- 8: erste Ausnehmung
- 9: zweite Ausnehmung
- 10: Spitze
- 11: abgeflachtes Ende
- 12: Lasche
- 13: Loch
- 14: Mittelsteg
- 15: Imaginäre Trennlinie/Sollbruchstelle/Perforationslinie/Sollbruchlinie

## Patentansprüche

1. Augenhöhlenabdeckgitter (1) mit einem Stege (4) ausbildenden perforierten Hauptkörper, der eine äußere Abschlusskante (6) aufweist, **dadurch gekennzeichnet, dass** mehrere längliche, durch einen Steg (4) von der Abschlusskante (6) getrennte und dort direkt angrenzende erste Ausnehmungen (5, 8) zumindest einem Abschnitt der Abschlusskante (6) folgend angeordnet sind.

2. Augenhöhlenabdeckgitter (1) nach Anspruch 1, **dadurch gekennzeichnet, dass** in Richtung des Zentrums des Gitters (1) eine oder mehrere zweite, längliche Ausnehmungen (5, 9) vorhanden sind, die die gleiche Ausrichtung wie die jeweils benachbarte erste Ausnehmung (5, 8) aufweisen.

3. Augenhöhlenabdeckgitter (1) nach Anspruch 2, **dadurch gekennzeichnet, dass** die innersten Ausnehmungen (5, 9) einer Bügeleisensohlenaußenkontur folgend oder angenähert dreieckig angeordnet sind.

4. Augenhöhlenabdeckgitter (1) nach Anspruch 2 oder 3, **dadurch gekennzeichnet, dass** der Hauptkörper (3) einen Erweiterungsbereich aufweist, in dem erste Ausnehmungen (5, 8) in einem spitzen Winkel zueinander angeordnet/ausgerichtet sind.

5. Augenhöhlenabdeckgitter (1) nach einem der Ansprüche 1 bis 4, **dadurch gekennzeichnet, dass** wenigstens eine radial vom Hauptkörper (2) abstehende Lasche (12) vorhanden ist, die zur Aufnahme von Befestigungsmitteln mit einem oder mehreren Löchern (13) versehen ist.

6. Augenhöhlenabdeckgitter (1) nach einem der Ansprüche 1 bis 5, **dadurch gekennzeichnet, dass** die im Hauptkörper (2) vorhandenen Ausnehmungen (5) überwiegend eine längliche Kontur aufweisen.

7. Augenhöhlenabdeckgitter (1) nach einem der Ansprüche 1 bis 6, **dadurch gekennzeichnet, dass** die Länge der Ausnehmung (5) mehr als 2,5 mal so groß wie deren Breite ist.

8. Augenhöhlenabdeckgitter (1) nach einem der Ansprüche 1 bis 7, **dadurch gekennzeichnet, dass** das Gitter (1) eine mehrfach gekrümmte Raumform einnimmt.

9. Augenhöhlenabdeckgitter (1) nach einem der Ansprüche 1 bis 8, **dadurch gekennzeichnet, dass** sich eine zweite Ausnehmung (5, 9) bis zu einem die Abschlusskante (6) ausformenden Steg (4) erstreckt.

10. Verfahren zum Herstellen eines Augenhöhlenabdeckgitters (1), vorzugsweise nach einem der Ansprüche 1 bis 9, **dadurch gekennzeichnet, dass** unter Nutzung von individuellen Patientendaten eine Negativform geschaffen wird, dann ein vorgefertigtes Rohstück mit ersten und zweiten länglichen Ausnehmungen (5, 8, 9) auf die Negativform aufgelegt wird, dann unter Aufbringung von Kraft/Druck und/oder Wärme das Rohstück an die Negativform angepasst wird, und das abgeformte Augenhöhlenabdeckgitter (1) von der Negativform abgenommen wird.
